# EUROPEAN PATENT SPECIFICATION

(11) **EP 3 139 907 B1**
(45) Date of publication and mention of the grant of the patent: **24.10.2018**
(21) Application number: 15722484.1
(22) Date of filing: 04.05.2015
(51) Int. Cl.: A61K 36/84, A61K 36/185, A61K 9/46, A61P 25/20

(54) **FAST DISSOLVING GRANULATE**
SCHNELLLÖSLICHES GRANULAT
GRANULÉ À DISSOLUTION RAPIDE

(30) Priority: 05.05.2014 EP 14167048
(43) Date of publication of application: 15.03.2017
(73) Proprietor: Sanofi-Aventis Deutschland GmbH, 65929 Frankfurt am Main (DE)
(72) Inventor: PLOHMANN, Bernd, 55216 Ingelheim am Rhein (DE); SCHEURING, Uwe, 55216 Ingelheim am Rhein (DE)
(74) Representative: Catillon, Marie
(86) International application number: PCT/EP2015/059667
(87) International publication number: WO 2015/169725

(56) References cited:
- WO-A2-2011/098394
- US-A1- 2007 196 348
- US-A1- 2009 304 602
- AXEL BRATTSTRÖM: "Scientific Evidence for a Fixed Extract Combination (Ze 91019) from Valerian and Hops traditionally used as a Sleep-inducing Aid", WIENER MEDIZINISCHE WOCHENSCHRIFT, SPRINGER-VERLAG, AT, vol. 157, no. 13-14, 1 July 2007 (2007-07-01), pages 367-370, XP019522532, ISSN: 1563-258X, DOI: 10.1007/S10354-007-0442-6
- SCHULZ ET AL: "Baldrian-Hopfen-Kombination ZE91019 bei Schlafstorungen uberlegen wirksam im Vergleich mit Baldrian allein bzw. mit Placebo", KOMPLEMENTAERE UND INTEGRATIVE MEDIZIN,, vol. 50, no. 3, 1 March 2009 (2009-03-01), pages 50-51, XP026034952, ISSN: 1863-8678, DOI: 10.1016/J.KIM.2009.02.008 [retrieved on 2009-03-27]

## Description

### BACKGROUND OF THE INVENTION

### 1. TECHNICAL FIELD

The invention relates to a fast dissolving granulate comprising a spray-dried methanolic or ethanolic extract of valerian root, a spray-dried methanolic extract of hop strobiles, one or more artificial sweeteners, one or more flavoring agents, for the treatment of sleep disorders.

### 2. PRIOR ART

Valerian and hop extracts are natural active principals and are widely used for treatment of sleep disorders. The assessment report (EMA/HMPC/215214/2008 of 6 May 2010) on "*Valeriana officinalis* L., radix and *Humulus lupulus* L., flos" of the European Medicines Agency (EMA) confirms the well-established use of coated tablets containing a dry methanolic or ethanolic extract of valerian root and a dry methanolic extract of hop strobiles for the treatment of mild forms of sleep disorders and/or nervous sleep.

Many patients suffering from mild forms of sleep disorders prefer to take a beverage which ameliorates the sleep disorder instead of taking a pill.

The German laying-out document ("Auslegeschrift") DE 1 073 153 of 14 January 1960 describes a process for the preparation of non-hygroscopic granules of extracts of valerian roots and hops. This process involves the granulation of powders of extracts of valerian roots and hops in the presence of milk powder in an aqueous alcohol followed by evaporation of the solvents at elevated temperatures.

However, such granules suffer from a loss of essential ethereal oils due to the evaporation and an abhorrent smell and taste.

US 2007/196348 describes fast dissolving powders compositions comprising valerian root and hop for the treatment of sleep disorders containing no methanolic or ethanolic extracts and no excipients.

The problem underlying the present invention was to provide a fast dissolving granule of a combined extract of valerian roots and of hop strobiles, which does not show the disadvantages of the known ones.

### SHORT SUMMARY OF THE INVENTION

Surprisingly this problem has been solved by the provision of a fast dissolving granulate comprising a spray-dried methanolic or ethanolic extract of valerian root, a spray-dried methanolic extract of hop strobiles, optionally a carrier, one or more artificial sweeteners, one or more flavoring agents, optionally an acidifier and water, and said granulate for use in the treatment of sleep disorders, wherein the said granulate is to be administered upon dissolution in water or an aqueous beverage shortly before bedtime.

A further aspect of the invention is a process of preparing a granulate according to the invention, which process comprises
(i) a) wetting a composition comprising a dry methanolic or ethanolic extract of valerian root, a dry methanolic extract of hop strobiles, optionally a carrier, one or more artificial sweeteners, one or more flavoring agents and optionally an acidifier with water, and b) spray-drying said aqueous solution to obtain a spray-dried granulate; or
(ii) combining a spray- dried methanolic or ethanolic extract of valerian root with a spray-dried methanolic extract of hop strobiles and optionally a carrier, and mixing one or more artificial sweeteners, one or more flavoring agents and optionally an acidifier with water with said spray-dried granulate.

Another aspect of the invention is a fast dissolving granulate comprising a spray-dried methanolic or ethanolic extract of valerian root having a native DER of 4-8 : 1, a spray-dried methanolic extract of hop strobiles having a native DER of 3-10 : 1, optionally a carrier, sucralose, one or more flavoring agents selected from the group consisting of chocolate, vanilla, cinnamon, blackberry and honey, optionally an acidifier selected from the group consisting of citric acid and ascorbic acid and water.

Furthermore, the invention relates to a kit of parts consisting essentially of:
(A) one or more sachets in a package containing a fast dissolving granulate according to claim 9 each with the same or different flavoring agents;
(B) a package leaflet, in which the opening of the sachet is explained, the amount of water or aqueous beverage, in which the granulate (A) is to be dissolved, is indicated, and the best timing for its administration for different patients is recommended.

Another aspect of the invention is a
fast dissolving granulate comprising
∘ a spray-dried methanolic or ethanolic extract of valerian root,
∘ a spray-dried methanolic extract of hop strobiles,
∘ optionally a carrier,
∘ one or more artificial sweeteners,
∘ one or more flavoring agents, and
∘ optionally an acidifier
mixed with water or an aqueous beverage for use in the treatment of sleep disorders to be administered shortly before bedtime to a person in need thereof.

### DETAILED DESCRIPTION OF THE INVENTION

The term "spray-drying" or "spray-dried" as used hereinbefore and herein below relates to the method of producing a dry powder or granulate from a liquid or slurry obtained from the raw extract of valerian root and/or hop strobiles with water and optionally other ingredients such as a carriers, artificial sweeteners, flavoring agents, optionally an acidifier, by rapidly drying with a hot gas. The extract of valerian root and the extract of hop strobiles may be spray-dried together or each on its own. As a rule air is used as the heated drying medium. All conventional spray dryers with atomizer or spray nozzle may be used to disperse the liquid or slurry into a controlled drop size spray. The most common of these are rotary disks and single-fluid high pressure swirl nozzles. Alternatively two-fluid or ultrasonic nozzles may be used. Drop sizes from 10 to 500 µm can be achieved, the most common applications are in the 100 to 200 µm diameter range.

The terms "spray-dried methanolic or ethanolic extract of valerian root", and "spray-dried methanolic extract of hop strobiles" as used hereinbefore and herein below relate to extracts of these plant parts for well-established use as referred to in EMA/HMPC/215214/2008 of 6 May 2010.
extracts of these plant parts for well-established use as referred to in EMA/HMPC/215214/2008 of 6 May 2010.

Preferably the spray-dried methanolic extract of valerian root is obtained by extraction with methanol 40-60 % (m/m), in particular 45-51% (m/m), having a drug native extract relationship (DER) of 4-8 : 1 and the spray-dried ethanolic extract of valerian root is obtained by extraction with ethanol 50-80 % (v/v), in particular about 70% (v/v), having a native DER of 4-7 : 1.

Preferably the spray-dried methanolic extract of hop strobiles is obtained by extraction with methanol 35-60 % (m/m), in particular 40-51% (m/m), having a native DER of 3-10 : 1.

Most preferred are the following combined extracts (A) and (B):
(A) valerian root (DER 4-8:1, methanol 45-51 % (m/m)) with hop strobiles (DER 3-10:1, methanol 40-51 % (m/m)), and
(B) valerian root (DER 4-7:1, ethanol 70 % (v/v)) with hop strobiles (DER 4-8:1, methanol 40 % (v/v)).

The above mentioned single extracts are commercially available from Herbal extract manufacturers (e.g. Finzelberg GmbH & Co. KG, Koblenzer Straße 48 - 56, D-56626 Andernach) authorized to produce pharmaceutical extracts as active ingredients of herbal medicinal products.

The ratio by weight between the extract of valerian roots and the extract of hop strobiles ranges from 10 : 1 to 4 : 1, preferably from 8 : 1 to 5 : 1.

The term "carrier" as used hereinbefore and herein below relates to any inert material which may be added to the extracts of active principles, sweeteners, flavoring agents, and optionally the acidifier, in order to improve the physical properties and appearance of the final granules. They may be added prior to the spray-drying processor thereafter. Preferred are those carriers, which are spray-drying aids, such as lactose, maltodextrin, or silica, (most preferred) lactose monohydrate and/or colloidal anhydrous silica.

The term "sweetener" as used hereinbefore and herein below includes both natural and artificial sweeteners. Preferred are natural sweeteners such as sucrose, sorbitol and stevia and artificial high-intensity sweeteners such aspartame, sucralose, neotame, acesulfame potassium, and saccharin. Most preferred are sucralose, sucrose and sorbitol, in particular sucralose.

The term "flavoring agent" as used hereinbefore and herein below includes natural, nature-identical and artificial flavoring agents. In principle the type of flavoring agent is not as critical. However, many fruity tasting or herb tasting agents do not suffice to mask the disgusting smell and taste of the valerian extract. Preferred are artificial flavoring agents such as vanilla, honey, cinnamon, blackberry and chocolate.

Such flavoring agents are commercially available from manufacturers (e.g. Givaudan International AG, Uberlandstrasse 138, CH-8600 Dübendorf, Symrise AG, Mühlenfeldstraße 1, D-37603 Holzminden or DöhlerGroup, Riedstraße, D-64295 Darmstadt) authorized to produce flavoring agents for dietary supplements or medicinal products.

The ratio by weight between the extract of valerian roots and the flavoring agent ranges from 5 : 1 to 1 : 1, preferably from 3.5 : 1 to 2.5 : 1.

In certain cases it may also be desirable to add colorants, in order to match the appearance of the resulting beverage having a specific flavor with the expectation of the user. For vanilla, honey and cinnamon taste a light yellow to red colorant may be added and for blackberry and chocolate taste a purple, brown or black colorant or cocoa may be added.

Such colorants are commercially available from manufacturers (e.g. DöhlerGroup, Riedstraße, D-64295 Darmstadt) authorized to produce colorants for beverages, dietary supplements or medicinal products.

The term "treatment of sleep disorders" as used hereinbefore and herein below relates to any forms of sleep disorders including problems to fall asleep despite great weariness or fatigue or problems to stay asleep during the night or finding a refreshing sleep. The fast dissolving granulate according to the invention provides relief of such sleep disorders and improves sleep latency and sleep quality.

The term "shortly before bedtime" as used hereinbefore and herein below relates to a time span of 15 to 120 minutes, preferably 30 to 90 minutes before going to bed. As a rule the patient will take the drinking solution obtained by mixing the fast dissolving granulates of the invention in water or an aqueous beverage shortly after dinner, preferably within 10 to 120, in particular 20 to 60 minutes thereafter.

The term "effective amount" as used hereinbefore and herein below relates to the amount the combined extracts which is needed to ameliorate the sleep disorder of the person in need thereof. Preferably a dose unit contains 200 to 700 mg, in particular 300 to 600 mg, most preferred about 350 or 500 mg of the spray dried extract of Valerian root (DER 4-8:1, methanol 45-51 % (m/m)) and 20 to 100 mg, in particular 50 to 80 mg, most preferred about 65 or 70 mg of the spray dried extract of Hop strobiles (DER 3-10:1, methanol 40-51 % (m/m)).

Most preferred are granulates which exhibit the following composition per 1 unit dose:

| **Component** | **Amount [mg]** |
|---|---|
| Valerian root (DER 4-7:1, ethanol 70 % (v/v)) | 300 - 600 |
| Hop strobiles (DER 4-8:1, methanol 40 % (v/v)) | 50 - 80 |
| Silica, colloidal anhydrous PH.EUR. | 15 - 30 |
| Lactose monohydrate PH.EUR | 100 - 150 |
| Sucralose | 5 - 25 |
| Artificial flavor | 150 - 250 |
| Citric acid anhydrous PH.EUR | 0 - 250 |

The resulting fast dissolving granulate is highly hygroscopic and is therefore preferably filled into sealed compound aluminum foil sachets.

In the following the invention shall be illustrated in form of exemplary formulations. However, the present invention is not limited to the described formulations, but other dosage forms and additives are possible.

### EXAMPLES

### Example A1

| **Component** | **Amount [mg]** | **Function** |
|---|---|---|
| Valerian root (DER 4-8:1, methanol 45-51 % (m/m)) | 500 | Active principle |
| Hop strobiles (DER 3-10:1, methanol 40-51 % (m/m)) | 65 | Active principle |
| Silica, colloidal anhydrous PH.EUR. | 21.2 | Spray-drying aid |
| Lactose monohydrate PH.EUR | 120 | Spray-drying aid |
| Sucralose | 15 | Sweetener |
| Artificial vanilla flavor | 200 | Flavor |

### Example A2

| **Component** | **Amount [mg]** | **Function** |
|---|---|---|
| Valerian root (DER 4-8:1, methanol 45-51 % (m/m)) | 500 | Active principle |
| Hop strobiles (DER 3-10:1, methanol 40-51 % (m/m)) | 65 | Active principle |
| Silica, colloidal anhydrous PH.EUR. | 21.2 | Spray-drying aid |
| Lactose monohydrate PH.EUR | 120 | Spray-drying aid |
| Sucralose | 15 | Sweetener |
| Artificial honey flavor | 200 | Flavor |

### Example A3

| **Component** | **Amount [mg]** | **Function** |
|---|---|---|
| Valerian root (DER 4-8:1, methanol 45-51 % (m/m)) | 500 | Active principle |
| Hop strobiles (DER 3-10:1, methanol 40-51 % (m/m)) | 65 | Active principle |
| Silica, colloidal anhydrous PH.EUR. | 21.2 | Spray-drying aid |
| Lactose monohydrate PH.EUR | 120 | Spray-drying aid |
| Sucralose | 15 | Sweetener |
| Artificial cinnamon flavor | 150 | Flavor |

### Example A4

| **Component** | **Amount [mg]** | **Function** |
|---|---|---|
| Valerian root (DER 4-8:1, methanol 45-51 % (m/m)) | 500 | Active principle |
| Hop strobiles (DER 3-10:1, methanol 40-51 % (m/m)) | 65 | Active principle |
| Silica, colloidal anhydrous PH.EUR. | 21.2 | Spray-drying aid |
| Lactose monohydrate PH.EUR | 120 | Spray-drying aid |
| Artificial blackberry flavor | 200 | Flavor |
| Citric acid anhydrous, PH.EUR | 200 | Acidifier |

### Example A5

| **Component** | **Amount [mg]** | **Function** |
|---|---|---|
| Valerian root (DER 4-8:1, methanol 45-51 % (m/m)) | 500 | Active principle |
| Hop strobiles (DER 3-10:1, methanol 40-51 % (m/m)) | 65 | Active principle |
| Silica, colloidal anhydrous PH.EUR. | 21.2 | Spray-drying aid |
| Lactose monohydrate PH.EUR | 120 | Spray-drying aid |
| Sucralose | 15 | Sweetener |
| Artificial chocolate flavor | 200 | Flavor |

### Example B1

| **Component** | **Amount [mg]** | **Function** |
|---|---|---|
| Valerian root (DER 4-7:1, ethanol 70 % (v/v)) | 350 | Active principle |
| Hop strobiles (DER 4-8:1, methanol 40 % (v/v)) | 70 | Active principle |
| Silica, colloidal anhydrous PH.EUR. | 21.2 | Spray-drying aid |
| Lactose monohydrate PH.EUR | 120 | Spray-drying aid |
| Sucralose | 15 | Sweetener |
| Artificial vanilla flavor | 200 | Flavor |

### Example B2

| **Component** | **Amount [mg]** | **Function** |
|---|---|---|
| Valerian root (DER 4-7:1, ethanol 70 % (v/v)) | 350 | Active principle |
| Hop strobiles (DER 4-8:1, methanol 40 % (v/v)) | 70 | Active principle |
| Silica, colloidal anhydrous PH.EUR. | 21.2 | Spray-drying aid |
| Lactose monohydrate PH.EUR | 120 | Spray-drying aid |
| Sucralose | 15 | Sweetener |
| Artificial honey flavor | 200 | Flavor |

### Example B3

| **Component** | **Amount [mg]** | **Function** |
|---|---|---|
| Valerian root (DER 4-7:1, ethanol 70 % (v/v)) | 350 | Active principle |
| Hop strobiles (DER 4-8:1, methanol 40 % (v/v)) | 70 | Active principle |
| Silica, colloidal anhydrous PH.EUR. | 21.2 | Spray-drying aid |
| Lactose monohydrate PH.EUR | 120 | Spray-drying aid |
| Sucralose | 15 | Sweetener |
| Artificial cinnamon flavor | 150 | Flavor |

### Example B4

| **Component** | **Amount [mg]** | **Function** |
|---|---|---|
| Valerian root (DER 4-7:1, ethanol 70 % (v/v)) | 350 | Active principle |
| Hop strobiles (DER 4-8:1, methanol 40 % (v/v)) | 70 | Active principle |
| Silica, colloidal anhydrous PH.EUR. | 21.2 | Spray-drying aid |
| Lactose monohydrate PH.EUR | 120 | Spray-drying aid |
| Sucralose | 15 | Sweetener |
| Artificial blackberry flavor | 200 | Flavor |
| Citric acid anhydrous PH.EUR | 200 | Acidifier |

### Example B5

| **Component** | **Amount [mg]** | **Function** |
|---|---|---|
| Valerian root (DER 4-7:1, ethanol 70 % (v/v)) | 350 | Active principle |
| Hop strobiles (DER 4-8:1, methanol 40 % (v/v)) | 70 | Active principle |
| Silica, colloidal anhydrous PH.EUR. | 21.2 | Spray-drying aid |
| Lactose monohydrate PH.EUR | 120 | Spray-drying aid |
| Sucralose | 15 | Sweetener |
| Artificial chocolate flavor | 200 | Flavor |

### Example C

The formulations of the examples A1 to A5 and B1 to B5 are filled into compound-aluminum foil sachets in portions to be dissolved in 150 to 250 ml of water (examples A1 to A4 and B1 to B4) or n 150 to 250 ml of hot milk (examples A5 and B5). The fast dissolving granules of the present examples may be upon dissolution in aqueous beverages or milk directly used for the treatment of heart sleep disorders in persons in need thereof.

The flavors of the resulting beverages were tested by 10 randomly selected volunteers (V1 to V10). They rated the results according to the following scheme:
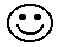 very good
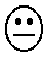 good
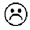 not acceptable
- not tested

The results of this taste assessment can be seen in the following table I:

**Table I: Taste Assessment**

| **Example** | **V1** | **V2** | **V3** | **V4** | **V5** | **V6** | **V7** | **V8** | **V9** | **V10** |
|---|---|---|---|---|---|---|---|---|---|---|
| **A1** | 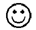 | 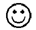 | 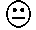 | 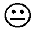 | 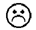 | 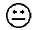 | 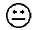 | 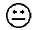 | 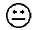 | 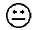 |
| **A2** | 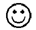 | 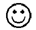 | 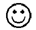 | 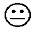 | 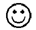 | 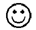 | 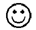 | 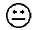 | 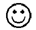 | 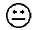 |
| **A3** | 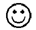 | 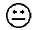 | 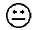 | 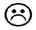 | 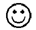 | 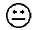 | 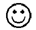 | 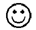 | 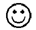 | 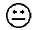 |
| **A4** | 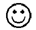 | 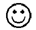 | 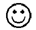 | 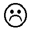 | 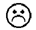 | 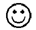 | 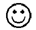 | 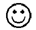 | 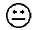 | 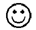 |
| **A5** | 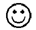 | 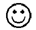 | 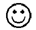 | 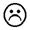 | 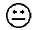 | 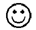 | 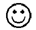 | 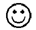 | 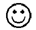 | 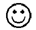 |
| **B1** | 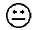 | 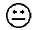 | 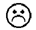 | 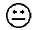 | 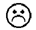 | 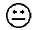 | 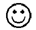 | 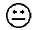 | 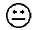 | **-** |
| **B2** | 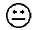 | 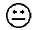 | 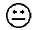 | 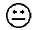 | 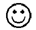 | 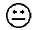 | 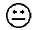 | 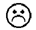 | 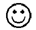 | **-** |
| **B3** | 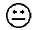 | 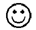 | 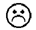 | 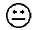 | 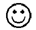 | 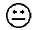 | 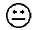 | 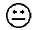 | 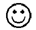 | **-** |
| **B4** | 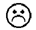 | 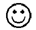 | 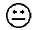 | 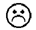 | 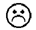 | 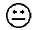 | 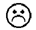 | 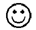 | 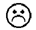 | **-** |
| **B5** | 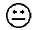 | 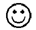 | 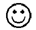 | 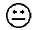 | 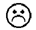 | 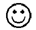 | 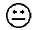 | 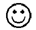 | 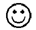 | **-** |

## Claims

1. A fast dissolving granulate comprising a spray-dried methanolic or ethanolic extract of valerian root, a spray-dried methanolic extract of hop strobiles, optionally a carrier, one or more sweeteners, one or more flavoring agents, optionally an acidifier and water.

2. The granulate according to the claim 1, wherein the dry methanolic extract of valerian root is obtained by extraction with methanol 40-60 % (m/m), in particular 45-51% (m/m), having a drug native extract relationship (DER) of 4-8 : 1 and the dry ethanolic extract of valerian root is obtained by extraction with ethanol 50-80 % (v/v), in particular about 70% (v/v), having a native DER of 4-7 : 1.

3. The granulate according to claims 1 or 2, wherein the dry methanolic extract of hop strobiles obtained by extraction with methanol 35-60 % (m/m), in particular 40-51% (m/m), having a native DER of 3-10 : 1.

4. The granulate according to claims 1 to 3, wherein the sweetener is selected from the group consisting of sucralose, sucrose and sorbitol.

5. The granulate according to claims 1 to 4, wherein the flavoring agent is selected from the group of natural, nature-identical or artificial flavors consisting of chocolate, vanilla, cinnamon, blackberry and honey.

6. The granulate according to claims 1 to 5, wherein the acidifier is selected from the group consisting of citric acid and ascorbic acid.

7. The granulate according to claims 1 to 6 for use in the treatment of sleep disorders wherein said granulate is mixed with water or an aqueous beverage to be administered shortly before bedtime to a person in need thereof.

8. A process of preparing a granulate according to claims 1 to 6, which process comprises
(i) a) wetting a composition comprising a dry methanolic or ethanolic extract of valerian root, a dry methanolic extract of hop strobiles, optionally a carrier, one or more artificial sweeteners, one or more flavoring agents and optionally an acidifier with water, and b) spray-drying said aqueous solution to obtain a spray-dried granulate; or
(ii) combining a spray- dried methanolic or ethanolic extract of valerian root with a spray-dried methanolic extract of hop strobiles and optionally a carrier, and mixing one or more artificial sweeteners, one or more flavoring agents and optionally an acidifier with water with said spray-dried granulate.

9. The process according to claim 8, wherein the spray-drying in step b) is carried out at an outlet air temperature of the spray-drier of between about 80 and 110°C.

10. A fast dissolving granulate comprising a spray-dried methanolic or ethanolic extract of valerian root having a native DER of 4-8 : 1, a spray-dried methanolic extract of hop strobiles having a native DER of 3-10 : 1, optionally a carrier, sucralose, one or more flavoring agents selected from the group consisting of chocolate, vanilla, cinnamon, blackberry and honey, optionally an acidifier selected from the group consisting of citric acid and ascorbic acid and water.

11. A kit of parts consisting essentially of:
(A) one or more sachets in a package containing a fast dissolving granulate according to claim 10 each with the same or different flavoring agents;
(B) a package leaflet, in which the opening of the sachet is explained, the amount of water or aqueous beverage, in which the granulate (A) is to be dissolved, is indicated, and the best timing for its administration for different patients is recommended.

## Patentansprüche

1. Schnelllösliches Granulat, umfassend einen sprühgetrockneten methanolischen oder ethanolischen Extrakt von Baldrianwurzel, einen sprühgetrockneten methanolischen Extrakt von Hopfenzapfen, gegebenenfalls einen Träger, einen oder mehrere Süßstoffe, einen oder mehrere Aromastoffe, gegebenenfalls ein Säuerungsmittel und Wasser.

2. Granulat gemäß Anspruch 1, wobei der trockene methanolische Extrakt von Baldrianwurzel durch Extraktion mit Methanol 40-60 % (m/m), insbesondere 45-51 % (m/m), mit einem nativen Droge-Extrakt-Verhältnis (DEV) von 4-8:1 erhalten ist, und der trockene ethanolische Extrakt von Baldrianwurzel durch Extraktion mit Ethanol 50-80 % (v/v), insbesondere etwa 70 % (v/v), mit einem nativen DEV von 4-7:1 erhalten ist.

3. Granulat gemäß Ansprüchen 1 oder 2, wobei der trockene methanolische Extrakt von Hopfenzapfen, der durch Extraktion mit Methanol 35-60 % (m/m), insbesondere 40-51 % (m/m), mit einem nativen DEV von 3-10:1 erhalten ist.

4. Granulat gemäß Ansprüchen 1 bis 3, wobei der Süßstoff ausgewählt ist aus der Gruppe bestehend aus Sucralose, Saccharose und Sorbit.

5. Granulat gemäß Ansprüchen 1 bis 4, wobei der Aromastoff ausgewählt ist aus der Gruppe von natürlichen, naturidentischen oder künstlichen Aromastoffen bestehend aus Schokolade, Vanille, Zimt, Brombeere und Honig.

6. Granulat gemäß Ansprüchen 1 bis 5, wobei das Säuerungsmittel ausgewählt ist aus der Gruppe bestehend aus Citronensäure und Ascorbinsäure.

7. Granulat gemäß Ansprüchen 1 bis 6 für die Verwendung bei der Behandlung von Schlafstörungen, wobei das Granulat zur Verabreichung kurz vor dem Schlafengehen an eine Person mit Bedarf daran mit Wasser oder einem wässrigen Getränk gemischt wird.

8. Verfahren zur Herstellung eines Granulats gemäß einem der Ansprüche 1 bis 6, wobei das Verfahren umfasst:
(i) a) Benetzen einer Zusammensetzung, die einen trockenen methanolischen oder ethanolischen Extrakt von Baldrianwurzel, einen trockenen methanolischen Extrakt von Hopfenzapfen, gegebenenfalls einen Träger, einen oder mehrere Süßstoffe, einen oder mehrere Aromastoffe und gegebenenfalls ein Säuerungsmittel umfasst, mit Wasser und b) Sprühtrocknen der wässrigen Lösung, um ein sprühgetrocknetes Granulat zu erhalten; oder
(ii) Kombinieren eines sprühgetrockneten methanolischen oder ethanolischen Extrakts von Baldrianwurzel mit einem sprühgetrockneten methanolischen Extrakt von Hopfenzapfen und gegebenenfalls einem Träger, und Mischen eines oder mehrerer künstlicher Süßstoffe, eines oder mehrerer Aromastoffe und gegebenenfalls eines Säuerungsmittels mit Wasser mit dem sprühgetrockneten Granulat.

9. Verfahren gemäß Anspruch 8, wobei das Sprühtrocknen bei Schritt b) bei einer Auslass-Lufttemperatur des Sprühtrockners von zwischen etwa 80 und 110 °C durchgeführt wird.

10. Schnellösliches Granulat, umfassend einen sprühgetrockneten methanolischen oder ethanolischen Extrakt von Baldrianwurzel mit einem nativen DEV von 4-8:1, einen sprühgetrockneten methanolischen Extrakt von Hopfenzapfen mit einem nativen DEV von 3-10:1, gegebenenfalls einen Träger, Sucralose, einen oder mehrere Aromastoffe ausgewählt aus der Gruppe bestehend aus Schokolade, Vanille, Zimt, Brombeere und Honig, gegebenenfalls ein Säuerungsmittel ausgewählt aus der Gruppe bestehend aus Citronensäure und Ascorbinsäure, und Wasser.

11. Satz von Teilen, bestehend im Wesentlichen aus:
(A) einem oder mehreren Sachets in einer Packung, die ein schnelllösliches Granulat gemäß Anspruch 10, jeweils mit dem gleichen oder verschiedenen Aromastoff(en), enthalten;
(B) Packungsbeilage, in der das Öffnen des Sachets erklärt wird, die Menge an Wasser oder wässrigem Getränk, worin das Granulat (A) gelöst werden soll, angegeben wird, und der beste Zeitpunkt für die Verabreichung für verschiedene Patienten empfohlen wird.

## Revendications

1. Granulé à dissolution rapide, comprenant un extrait méthanolique ou éthanolique séché par pulvérisation de racines de centranthe, un extrait méthanolique séché par pulvérisation de strobiles de houblon, éventuellement un véhicule, un ou plusieurs édulcorants, un ou plusieurs agents aromatisants, éventuellement un acidifiant et de l'eau.

2. Granulé selon la revendication 1, dans lequel l'extrait méthanolique sec de racines de centranthe est obtenu par extraction par du méthanol à 40-60% (m/m), en particulier 45-51% (m/m), ayant un rapport du médicament à l'extrait natif (DER) de 4-8:1 et l'extrait éthanolique sec de racines de centranthe est obtenu par extraction par de l'éthanol à 50-80% (v/v), en particulier environ 70% (v/v), ayant un DER natif de 4-7:1.

3. Granulé selon les revendications 1 ou 2, dans lequel l'extrait méthanolique sec de strobiles de houblon obtenu par extraction par du méthanol à 35-60% (m/m), en particulier 40-51% (m/m), ayant un DER natif de 3-10:1.

4. Granulé selon les revendications 1 à 3, dans lequel l'édulcorant est choisi dans le groupe constitué par le sucralose, le saccharose et le sorbitol.

5. Granulé selon les revendications 1 à 4, dans lequel l'agent aromatisant est choisi dans le groupe constitué par les arômes naturels, identiques aux naturels ou artificiels constitués par le chocolat, la vanille, la cannelle, la mûre et le miel.

6. Granulé selon les revendications 1 à 5, dans lequel l'acidifiant est choisi dans le groupe constitué par l'acide citrique et l'acide ascorbique.

7. Granulé selon les revendications 1 à 6, pour une utilisation dans le traitement des troubles du sommeil, où ledit granulé est mélangé avec de l'eau ou une boisson aqueuse, pour être administré, peu de temps avant l'heure du coucher, à une personne en ayant besoin.

8. Procédé de préparation d'un granulé selon les revendications 1 à 6, lequel procédé comprend
(i) a) l'humidification d'une composition comprenant un extrait méthanolique ou éthanolique sec de racines de centranthe, un extrait méthanolique sec de strobiles de houblon, éventuellement un véhicule, un ou plusieurs édulcorants artificiels, un ou plusieurs agents aromatisants, et éventuellement un acidifiant, par de l'eau et b) le séchage par pulvérisation de ladite solution aqueuse afin d'obtenir un granulé séché par pulvérisation ; ou
(ii) la combinaison d'un extrait méthanolique ou éthanolique de racines de centranthe séché par pulvérisation avec un extrait méthanolique de strobiles de houblon séché par pulvérisation, et éventuellement un véhicule, et le mélange d'un ou plusieurs édulcorants artificiels, d'un ou plusieurs agents aromatisants et éventuellement d'un acidifiant avec de l'eau, avec ledit granulé séché par pulvérisation.

9. Procédé selon la revendication 8, dans lequel le séchage par pulvérisation dans l'étape b) est effectué à une température d'air de sortie du séchoir-atomiseur comprise entre environ 80 et 110°C.

10. Granulé à dissolution rapide, comprenant un extrait méthanolique ou éthanolique de racines de centranthe séché par pulvérisation ayant un DER natif de 4-8:1, un extrait méthanolique de strobiles de houblon séché par pulvérisation ayant un DER natif de 3-10:1, éventuellement un véhicule, du sucralose, un ou plusieurs agents aromatisants choisis dans le groupe constitué par le chocolat, la vanille, la cannelle, la mûre et le miel, éventuellement un acidifiant choisi dans le groupe constitué par l'acide citrique et l'acide ascorbique et de l'eau.

11. Kit de parties, constitué essentiellement
(A) d'un ou plusieurs sachets dans un emballage contenant un granulé à dissolution rapide selon la revendication 10 chacun avec des agents aromatisants identiques ou différents ;
(B) une notice d'emballage, où on explique l'ouverture du sachet, où on indique la quantité d'eau ou de boisson aqueuse dans laquelle le granulé (A) doit être dissous, et où on conseille le meilleur moment pour son administration à différents patients.
